# EUROPÄISCHE PATENTSCHRIFT

(11) **EP 0 612 710 B1**
(45) Veröffentlichungstag und Bekanntmachung des Hinweises auf die Patenterteilung: **06.11.1996**
(21) Anmeldenummer: 94200073.8
(22) Anmeldetag: 13.01.1994
(51) Int. Cl.: C07C 29/149, C07C 31/125

(54) **Verfahren zum Regeln der Menge am gebrauchtem Katalysator, die in eine Hydriereinrichtung zum Erzeugen von Fettalkoholen aus Fettsäuren und/oder Fettsäurederivaten zurückgeführt wird**
Method of controlling the amount of used catalyst to be recycled to a hydrogenation reactor, used to produce fatty alcohols from fatty acids, or their derivatives
Procédé de régulation du catalyseur usé, ramené à un réacteur d'hydrogénation, utilisé pour la production d'alcools gras à partir d'acides gras

(30) Priorität: 13.02.1993 DE 4304420
(43) Veröffentlichungstag der Anmeldung: 31.08.1994
(73) Patentinhaber: METALLGESELLSCHAFT Aktiengesellschaft, 60015 Frankfurt (DE)
(72) Erfinder: Stönner, Hans-Martin, D-65760 Eschborn (DE); Buchold, Henning, Dr., D-63452 Hanau (DE)

(56) Entgegenhaltungen:
- CH-A- 326 946
- DE-A- 3 221 307
- DE-B- 1 112 056

## Beschreibung

Die Erfindung betrifft ein Verfahren zum Regeln der Menge an gebrauchtem Katalysator, die in eine Hydriereinrichtung zurückgeführt wird, wobei man der Hydriereinrichtung Fettsäuren und/oder Fettsäurederivate, Wasserstoff, frischen Katalysator und eine gebrauchten Katalysator enthaltende Dispersion zuführt und bei Temperaturen im Bereich von 100 bis 400°C und Drücken im Bereich von 20 bis 400 bar einen Produktstrom erzeugt, der Fettalkohole und gebrauchten Katalysator enthält.

Aus den US-Patenten 2 750 429, 4 259 536 und 4 482 766 ist die Herstellung von Fettalkoholen durch Hydrieren von Fettsäuren und/oder Fettsäurederivaten bekannt. Man verwendet dabei einen sehr feinteiligen Metallkatalysator, z.B. auf Kupferbasis, der üblicherweise Korngrößen im Bereich von 1 bis 100 µm aufweist. Der gebrauchte Katalysator befindet sich auch im fettalkoholreichen Produktstrom, er wird daraus abgetrennt und mindestens teilweise wieder zur Hydrierung zurückgeführt. Derivate, die anstelle der Fettsäuren der Hydrierung aufgegeben werden können, sind in bekannter Weise z.B. Fettsäureester oder Glyzeride.

Der Erfindung liegt die Aufgabe zugrunde, die bekannten Verfahren weiterzuentwickeln und die Menge des gebrauchten Katalysators, die in die Hydrierung zurückgeführt wird, auf einfache Weise zu regeln. Erfindungsgemäß geschieht dies beim eingangs genannten Verfahren dadurch, daß man den Produktstrom teilweise im Kreislauf führt, aus dem Kreislauf einen ersten Teilstrom mit regelbarer Menge abzieht und durch eine Trenneinrichtung leitet, aus der Trenneinrichtung fettalkoholreiches Produkt und getrennt davon eine gebrauchten Katalysator enthaltende Dispersion abzieht und die Dispersion in die Hydriereinrichtung zurückführt, daß man aus dem Kreislauf einen zweiten Teilstrom mit regelbarer Menge abzieht, den gebrauchten Katalysator abtrennt und aus dem Verfahren entfernt, und daß man zum Vergrößern der Menge an der Hydriereinrichtung zugeführtem, gebrauchtem Katalysator die Menge des zweiten Teilstroms verringert, und daß man zum Verringern der Menge an der Hydriereinrichtung zugeführtem, gebrauchtem Katalysator die Menge des zweiten Teilstroms vergrößert. Als Trenneinrichtung kommt z.B. ein rückspülbares Filter oder eine Hochleistungszentrifuge infrage.

Im Normalbetrieb, wenn die Mengen des ersten und zweiten Teilstroms konstant gehalten werden, werden kleine Schwankungen der Katalysatorkonzentration in der Hydriereinrichtung von selbst reguliert. Es wird nämlich bei einem Absinken der Katalysatorkonzentration auch nur eine verringerte Menge an Katalysator über den zweiten Teilstrom aus dem Verfahren entfernt und andererseits wird beim Ansteigen der Katalysatorkonzentration mehr Katalysator über den zweiten Teilstrom abgezogen. Auf diese Weise wird eine Selbststabilisierung der Katalysatorkonzentration erreicht, ohne daß zusätzlich frischer Katalysator aufgewendet werden muß.

Zweckmäßigerweise trennt man den gebrauchten Katalysator aus dem zweiten Teilstrom durch Zentrifugieren oder Dekantieren ab. Dies geschieht mit Vorteil ohne Zugabe von Filterhilfsmitteln und führt zu einer flüssigkeitsarmen Phase mit 50 bis 80 Gew.% Feststoffgehalt. Diese den verbrauchten Katalysator enthaltende Phase kann ohne Probleme deponiert werden.

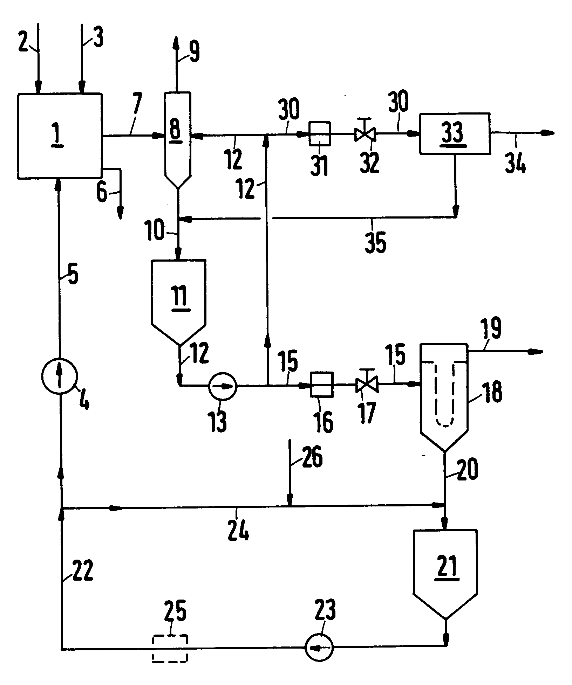

Ausgestaltungsmöglichkeiten des Verfahrens werden mit Hilfe der Zeichnung erläutert.

Das zu hydrierende Material, z.B. ein Fettsäuregemisch, wird der Hydrierung (1) durch die Leitung (2) in dosierter Menge zugeführt. Wasserstoff wird in der Leitung (3) zugespeist. Über eine Hochdruck-Dosierpumpe (4) wird durch die Leitung (5) eine Dispersion herangeführt, die gebrauchten Katalysator enthält. Der Produktstrom, der Fettalkohole und gebrauchten Katalysator enthält, wird in der Leitung (7) abgezogen und zunächst in einen Entspannungsbehälter (8) geleitet. Entstehendes Reaktionswasser wird über die Leitung (6) abgeschieden. Wasserstoffhaltiges Entspannungsgas zieht man in der Leitung (9) ab und das entspannte, flüssige Produkt gelangt in der Leitung (10) zu einem Zwischenbehälter (11). Durch die Kreislaufleitung (12) und die Kreislaufpumpe (13) wird der Produktstrom teilweise zum Entspannungsbehälter (8) zurückgeführt.

Einen ersten Teilstrom des Produktstroms der Kreislaufleitung (12) zieht man in der Leitung (15) ab, führt ihn durch einen Durchflußmesser (16) und ein Regelventil (17) und gibt ihn dem Schlauchfilter (18) auf. Im Filter (18) wird ein fettalkoholreiches, feststofffreies Produkt gewonnen, das man in der Leitung (19) abzieht. Dieses Produkt kann einer nicht dargestellten Feinstreinigung unterzogen werden.

Im Filter (18) fällt eine gebrauchten Katalysator enthaltende Dispersion an, die man durch die Leitung (20) zunächst zu einem zwischenbehälter (21) führt, bevor man sie durch die Leitung (22) über die Vordruckpumpe (23) teilweise in die Leitung (5) einspeist. Zweckmäßigerweise leitet man einen Teil der Dispersion durch die Leitung (24) kontinuierlich im Kreislauf zurück zum Zwischenbehälter (21).

Frischer Katalysator kommt aus der Leitung (26) und wird in dosierter Menge der Dispersion der Leitung (24) zugemischt. Auf diese Weise wird eine homogene Verteilung des frischen Katalysators in der Dispersion erreicht.

Aus der Kreislaufleitung (12) wird ein zweiter Teilstrom des flüssigen Produkts durch die Leitung (30) abgezweigt und über einen Durchflußmesser (31) und ein Regelventil (32) einem Dekanter (33) zugeführt. An die Stelle des Dekanters (33) kann z.B. auch eine Zentrifuge treten. Der Dekanter (33) gibt in der Leitung (34) eine katalysatorreiche Phase ab, die üblicherweise zu 50 bis 80 Gew.% aus Feststoff besteht. Diese Phase kann problemlos deponiert werden. Der im Dekanter (33) anfallende Fettalkohol wird durch die Leitung (35) dem Produktstrom der Leitung (10) zugemischt.

Die Produktströme, die durch die Leitungen (15) und (30) aus der Kreislaufleitung (12) abgezogen werden, sind variabel und unterschiedlich groß, das Mengenverhältnis liegt üblicherweise bei 100 : 1 bis 5 : 1. Will man die Menge des der Hydriereinrichtung zugeführten gebrauchten Katalysators erhöhen, so verringert man die Menge des durch die Leitung (30) fließenden zweiten Teilstroms durch Betätigen des Regelventils (32). Dadurch sinkt vorübergehend die Menge des durch die Leitung (34) abgezogenen Katalysators, wodurch der Katalysatorgehalt in der Dispersion in den Leitungen (12), (15) und (20) ansteigt. Dies führt zu einem höheren Katalysatorgehalt sowohl in der Leitung (22) als auch in der Leitung (5), die den gebrauchten Katalysator zusammen mit dem frischen in die Hydriereinrichtung (1) zurückführt. Im schließlich erreichten neuen Beharrungszustand wird wiederum durch die Leitung (34) genauso viel Katalysator abgezogen, wie man durch die Leitung (26) zuführt. Um die Pumpbarkeit der Dispersion in der Leitung (22) sicherzustellen, kann es zweckmäßig sein, ein Dichtemeßgerät (25) vorzusehen, das gestrichelt angedeutet ist.

In analoger Weise erreicht man das Verringern der Menge an der Hydriereinrichtung (1) zugeführtem gebrauchtem Katalysator dadurch, daß man die Menge des in der Leitung (30) abgezogenen zweiten Teilstroms vergrößert. Vorübergehend steigt dadurch die Menge an durch die Leitung (34) abgeführtem Katalysator und gleichzeitig sinkt der Katalysatorgehalt in den Leitungen (12), (15) und (20). Dies führt schließlich zu einem geringeren Katalysatorgehalt in der Dispersion der Leitung (5). Bei diesen Regelungsmaßnahmen kann die Zufuhr an frischem Katalysator durch die Leitung (26) ungeändert bleiben.

### Beispiel

In einer der Zeichnung entsprechenden Anlage mit einer Hydrierung (1) gemäß US-Patent 4 259 536 werden pro Stunde aus 4334 kg Fettsäure aus Palmkernöl, die in der Leitung (2) herangeführt werden, 4081 kg Rohfettalkohol erzeugt, die man in der Leitung (19) abzieht. Die Fettsäure (im wesentlichen C₁₂-C₁₈) weist eine Verseifungszahl von 252 mg KOH/g auf. Der Hydrierung (1) werden durch die Leitung (3) 87 kg/h Frischwasserstoff zugeführt; die Menge des in der Zeichnung nicht dargestellten Kreislauf-Wasserstoffs beträgt 1653 kg/h. Der Hydrierreaktor arbeitet bei einem Druck von 296 bar und einer Temperatur von 265°C. Man verwendet einen pulverförmigen kupferhaltigen Katalysator der Korngröße 1 bis 50 µm von der Firma Süd-Chemie, München, mit der Bezeichnung G99B-O. Pro Liter Einsatz Fettsäure werden der Anlage 3,1 g Katalysator durch die Leitung (26) kontinuierlich zugerührt und die gleiche Menge an Katalysator wird in der Leitung (34) aus dem Verfahren entfernt. Weitere Verfahrensdaten, die teilweise berechnet sind, ergeben sich aus nachfolgender Tabelle, Spalte A.

| | A | B |
|---|---|---|
| Katalysator-Gehalt in Leitung (12) | 4 Gew.% | 6 Gew.% |
| Gewichtsverhältnis von frischem zu gebrauchtem Katalysator in Leitung (5) | 14,84 | 22,85 |
| Produktmenge in Leitung (30) | 420 l/h | 280 l/h |
| Verseifungszahl des Produkts in Leitung (19) in mg KOH/g | 5 | 5 |
| Nebenprodukte, insbesondere Kohlenwasserstoffe, im Produkt der Leitung (19) | 0,30 Gew.% | 0,22 Gew.% |

Wenn man die Produktmenge der Leitung (30) auf 280 l/h verringert, steigt der Katalysator-Gehalt in dieser Leitung von 4 Gew.% allmählich auf 6 Gew.%, wobei man nach ca. 400 Betriebsstunden asymptotisch diesen Wert erreicht. Dabei wird in dieser Zeit die Temperatur im Hydrierreaktor, bei unverändertem Druck, schrittweise auf 258°C verringert, damit im Rohfettalkohol der Leitung (19) die Verseifungszahl konstant bleibt. Weitere Daten des neuen, stationären Zustands ergeben sich aus obiger Tabelle in Spalte B.

Man kann auch umgekehrt verfahren, von den Daten der Spalte B ausgehen, und durch Erhöhen der Produktmenge in der Leitung (30) auf 420 l/h sich schließlich asymptotisch der Katalysator-Konzentration in der Leitung (30) von 4 Gew.% nähern. In diesem Fall wird die Reaktortemperatur zügig von 258°C auf 265°C erhöht, so daß die Verseifungszahl im Rohfettalkohol der Leitung (19) nicht über 5 mg KOH/g ansteigt.

Bei den obigen Daten geht man von konstanter Zufuhr von 3,1 g frischem Katalysator durch die Leitung (26) pro Liter Einsatz-Fettsäure der Leitung (2) aus. Wenn man diese Frischkatalysator-Zufuhr auf 6,2 g/l verdoppelt, muß man die in der Leitung (30) abgezogene Produktmenge ebenfalls verdoppeln, wenn die Konzentration des Katalysators in den Leitungen (12), (15) und (30) nach einiger Betriebszeit wieder auf den ursprünglichen Wert zurückgeführt werden soll. Ersichtlich wird so der Katalysatorhaushalt in der Hydrieranlage auf einfache Weise geregelt, ohne daß es zu unzulässigen oder unerwünschten Katalysator-Konzentrationen kommt.

## Patentansprüche

1. Verfahren zum Regeln der Menge an gebrauchtem Katalysator, die in eine Hydriereinrichtung zurückgeführt wird, wobei man der Hydriereinrichtung Fettsäuren und/oder Fettsäurederivate, Wasserstoff, frischen Katalysator und eine gebrauchten Katalysator enthaltende Dispersion zuführt und bei Temperaturen im Bereich von 100 bis 400°C und Drücken im Bereich von 20 bis 400 bar einen Produktstrom erzeugt, der Fettalkohole und gebrauchten Katalysator enthält, dadurch gekennzeichnet, daß man den Produktstrom teilweise im Kreislauf (12) führt, aus dem Kreislauf einen ersten Teilstrom (15) mit regelbarer Menge abzieht und durch eine Trenneinrichtung (18) leitet, aus der Trenneinrichtung fettalkoholreiches Produkt (19) und getrennt davon eine gebrauchten Katalysator enthaltende Dispersion (20) abzieht und die Dispersion in die Hydriereinrichtung zurückführt, daß man aus dem Kreislauf einen zweiten Teilstrom (30) mit regelbarer Menge abzieht, den gebrauchten Katalysator abtrennt und aus dem Verfahren entfernt (34) und daß man zum Vergrößern der Menge an der Hydriereinrichtung zugeführtem gebrauchtem Katalysator die Menge des zweiten Teilstroms verringert und daß man zum Verringern der Menge an der Hydriereinrichtung zugeführtem gebrauchtem Katalysator die Menge des zweiten Teilstroms vergrößert.

2. Verfahren nach Anspruch 1, dadurch gekennzeichnet, daß man der gebrauchten Katalysator enthaltenden Dispersion frischen Katalysator (26) zumischt.

3. Verfahren nach Anspruch 1 oder 2, dadurch gekennzeichnet, daß man den gebrauchten Katalysator aus dem zweiten Teilstrom durch Zentrifugieren oder Dekantieren abtrennt.

4. Verfahren nach Anspruch 1 oder 3, dadurch gekennzeichnet. daß man beim Abtrennen des gebrauchten Katalysators aus dem zweiten Teilstrom einen fettalkoholhaltigen Flüssigkeitsstrom (35) erhält, den man dem Produktstrom zumischt.

## Claims

1. A method for controlling the rate of spent catalyst which is recycled into a hydrogenation means, wherein fatty acids and/or fatty acid derivatives, hydrogen, fresh catalyst and a dispersion containing spent catalyst are supplied to the hydrogenation means and at temperatures in the range of 100 to 400°C and pressures in the range of 20 to 400 bar a product stream is produced which contains fatty alcohols and spent catalyst, characterised in that the product stream is partly circulated (12), a first partial stream (15) is withdrawn from the circuit at a controllable rate and is passed through a separation means (18), product (19) rich in fatty alcohols and, separately therefrom, a dispersion (20) containing spent catalyst are withdrawn from the separation means, and the dispersion is recycled into the hydrogenation means, that a second partial stream (30) is removed from the circuit at a controllable rate, the spent catalyst is separated off and removed from the process (34) and that in order to increase the rate of spent catalyst supplied to the hydrogenation means the rate of the second partial stream is decreased and that in order to decrease the rate of spent catalyst supplied to the hydrogenation means the rate of the second partial stream is increased.

2. A method according to Claim 1, characterised in that fresh catalyst (26) is admixed to the dispersion containing spent catalyst.

3. A method according to Claim 1 or 2, characterised in that the spent catalyst is separated from the second partial stream by centrifuging or decanting.

4. A method according to Claim 1 or 3, characterized in that when separating off the spent catalyst from the second partial stream a liquid stream (35) containing fatty alcohol is obtained, which is admixed to the product stream.

## Revendications

1. Procédé de réglage de la quantité de catalyseur usé qui est retourné à un dispositif d'hydrogénation, qui consiste à envoyer au dispositif d'hydrogénation des acides gras et/ou des dérivés d'acides gras, de l'hydrogène, du catalyseur frais et une dispersion contenant du catalyseur usé et à produire à des températures de l'ordre de 100 à 400°C et sous des pressions de l'ordre de 20 à 400 bars un courant de produit qui contient des alcools gras et du catalyseur usé, caractérisé en ce qu'il consiste à faire passer le courant de produit en partie en circuit fermé (12), à soutirer du circuit en une quantité pouvant être réglée un premier courant partiel (15) et à le faire passer dans un dispositif de séparation (18), à soutirer du dispositif de séparation du produit (19) riche en alcool gras et indépendamment de celui-ci une dispersion (20) contenant du catalyseur usé et à retourner la dispersion au dispositif d'hydrogénation, à soutirer du circuit dans une quantité pouvant être réglée un deuxième courant partiel (30), à séparer le catalyseur usé et à l'éliminer (34) du procédé et, pour augmenter la quantité du catalyseur usé envoyé au dispositif d'hydrogénation, a diminuer la quantité du deuxième courant partiel et, pour diminuer la quantité de catalyseur usé envoyé au dispositif d'hydrogénation, à augmenter la quantité du deuxième courant partiel.

2. Procédé suivant la revendication 1, caractérisé en ce qu'il consiste à mélanger du catalyseur (26) frais à la dispersion contenant le catalyseur usé.

3. Procédé suivant la revendication 1 ou 2, caractérisé en ce qu'il consiste à séparer le catalyseur usé du deuxième courant partiel par centrifugation ou par décantation.

4. Procédé suivant la revendication 1 ou 3, caractérisé en ce qu'il consiste à obtenir lors de la séparation du catalyseur usé du deuxième courant partiel, un courant (35) de liquide contenant des alcools gras que l'on mélange au courant de produit.
